# EUROPÄISCHE PATENTSCHRIFT

(11) **EP 2 629 727 B1**
(45) Veröffentlichungstag und Bekanntmachung des Hinweises auf die Patenterteilung: **17.01.2018**
(21) Anmeldenummer: 11773683.5
(22) Anmeldetag: 13.10.2011
(51) Int. Cl.: A61F 13/49, A61F 13/15

(54) **VERFAHREN ZUR HERSTELLUNG EINER INKONTINENZWEGWERFWINDEL MIT KONTURIERTEN WINDELSEITENTEILEN**
METHOD FOR PRODUCING A DISPOSABLE INCONTINENCE DIAPER WITH CONTOURED DIAPER SIDE PARTS
PROCÉDÉ DE FABRICATION D'UNE COUCHE D'INCONTINENCE JETABLE COMPORTANT DES PARTIES LATÉRALES DE COUCHE PROFILÉES

(30) Priorität: 21.10.2010 DE 102010049171
(43) Veröffentlichungstag der Anmeldung: 28.08.2013
(73) Patentinhaber: Paul Hartmann AG, 89522 Heidenheim (DE)
(72) Erfinder: MALOWANIEC, Krzysztof, Daniel, 89522 Heidenheim (DE)
(86) Internationale Anmeldenummer: PCT/EP2011/005145
(87) Internationale Veröffentlichungsnummer: WO 2012/052134

(56) Entgegenhaltungen:
- EP-A1- 2 238 955
- WO-A1-2004/052260
- WO-A1-2005/016200
- WO-A1-2012/003965
- JP-A- 4 261 655
- US-A1- 2005 148 965

## Beschreibung

Die Erfindung betrifft ein Verfahren zum Herstellen einer Vielzahl von einen Windelhauptteil und daran angefügte vordere und hintere Windelseitenteile aufweisenden Inkontinenzwegwerfwindeln des offenen Typs für Erwachsene. Derartige Inkontinenzwegwerfwindeln sind bekannt und weisen einen Hauptteil, bestehend aus einem Vorderbereich, einem Rückenbereich und einem in Längsrichtung dazwischen liegenden zwischen den Beinen eines Benutzers zu liegen kommenden Schrittbereich auf, wobei der Hauptteil meist bereits einen Absorptionskern umfasst, und mit vier an den Rückenbereich und an den Vorderbereich beidseits angefügten, voneinander separaten Windelseitenteilen, welche sich in Querrichtung über seitliche Längsränder des Hauptteils hinaus erstrecken und den Vorderbereich und den Rückenbereich im angelegten Zustand des Artikels miteinander verbinden.

Die Windelseitenteile werden vorzugsweise im Cut & Place-Verfahren direkt, beidseitig an den Windelhauptteil, also das Chassis des Hygieneartikels angefügt. Diese Fertigungstechnologie erlaubt es, die Windelseitenteile aus einem anderen Rohmaterial zu fertigen als den mittleren Windelhauptteil des Hygieneartikels. Beispielsweise können die Windelseitenteile luftdurchlässig ausgeführt werden, wohingegen der mittlere Windelhauptteil im Wesentlichen feuchtigkeitsundurchlässig ausgebildet werden kann.

Die aus der Fertigungssicht effizienteste und einfachste sowie kostengünstigste Form der Windelseitenteile ist die rechteckige Form. Sie erlaubt bei der Herstellung den Transport der die Windelseitenteile bildenden Materialien in Form einer endlosen Flachmaterialbahn, von der dann die Windelseitenteile quer zur Maschinenrichtung abgetrennt werden. Ein Schnittabfall ist hier praktisch nicht gegeben.

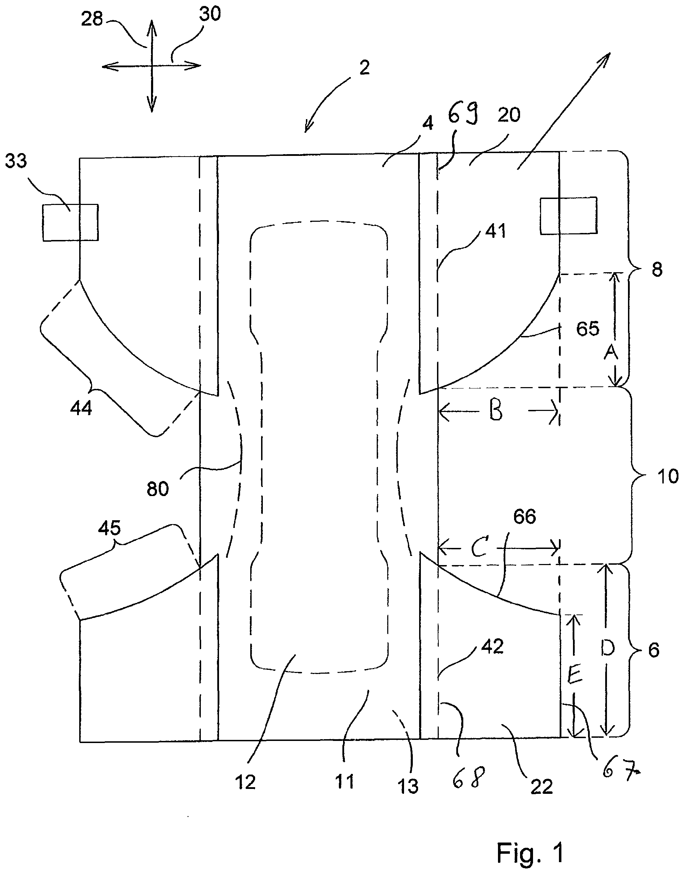

Es hat sich jedoch gezeigt, dass insbesondere bei der Ausbildung der Windelseitenteile in der ansonsten vorteilhaften Rechteckform beim Anlegen und Tragen des Hygieneartikels mitunter das Problem besteht, dass die angefügten Windelseitenteile im Bereich der seitlichen Längsränder des Windelhauptteils einreißen können. Es hat sich nämlich gezeigt, dass Anwender beim Anlegen des Hygieneartikels geneigt sind, einen zur Quer- und Längsrichtung des Hygieneartikels schrägen Zug auf die im Falle von Inkontinenzartikeln für Erwachsene extrem weit in Quer- und Längsrichtung ausladenden Windelseitenteile auszuüben, was in Figur 1 mit einem schräg nach oben gerichteten Pfeil angedeutet ist. Es kann solchenfalls vorkommen, dass Windelseitenteile entlang der seitlichen Längsränder des Windelhauptteils einreißen, wobei der Riss ausgehend von dem dem Schrittbereich zugewandten Querrand des Windelseitenteils ausgeht. Die DE102010026643 (noch unveröffentlicht) schlägt daher vor, die hinteren Windelseitenteile mit einer Konturierung zu versehen, derart, dass ein unterer Rand der hinteren Windelseitenteile kurvenförmig ausgebildet ist, dass der untere Rand einen konvexen Abschnitt aufweist, dass das Verhältnis der Länge A des unteren Randes eines hinteren Windelseitenteils zur der Breite B des unteren Randes eines hinteren Windelseitenteils 0,4-0,9 beträgt und das Verhältnis der Längserstreckung E eines Extrempunktes P des konvexen Abschnittes zu dessen Quererstreckung F 0,15-0,80 beträgt. Die DE102010026643 zeigt in einer Ausführungsform auch bereits vordere Windelseitenteile mit konkaver Beinöffnungskontur.

Der vorliegenden Erfindung liegt nun die Aufgabe zugrunde, ein kostengünstig und prozesstechnisch vorteilhaft realisierbares Verfahren zum Herstellen von derartigen oder ähnlichen Inkontinenzwegwerfwindeln zu schaffen.

Diese Aufgabe wird erfindungsgemäß gelöst durch ein Verfahren mit den folgenden Verfahrensschritten:
- Zuführen und Fördern einer endlosen Windelhauptteilbahn in einer ersten Längsrichtung zu einer Trenn- und Applikationsstation
- Zuführen einer endlosen Windelseitenteilbahn zu der Trenn- und Applikationsstation, wobei an der Trenn- und Applikationsstation aufeinander folgende erste und zweite doppelnutzige Längsabschnitten mit zumindest bereichsweise gekrümmten Rändern von der Windelseitenteilbahn abgetrennt werden und an ersten und zweiten Bereichen der Windelhauptteilbahn angefügt werden, wobei die ersten und zweiten Bereiche jeweils einen Hüftbereich zweier in Längsrichtung aufeinander folgender und aneinander angrenzender Windelhauptteile umfassen
- Vereinzeln der Inkontinenzwegwerfwindeln durch Trennen der Windelhauptteilbahn quer zur ersten Längsrichtung, wobei das Trennen durch die Längsabschnitte hindurchgeführt wird, derart, dass ein erster Teilabschnitt zumindest eines ersten jeweiligen Längsabschnitts ein hinteres Windelseitenteil einer ersten Inkontinenzwegwerfwindel bildet und ein zweiter Teilabschnitt des jeweiligen ersten Längsabschnitts ein vorderes oder hinteres Windelseitenteil einer unmittelbar angrenzenden zweiten Inkontinenzwegwerfwindel bildet, und dass ein erster Teilabschnitt eines zweiten jeweiligen Längsabschnitts ein vorderes Windelseitenteil einer Inkontinenzwegwerfwindel bildet und ein zweiter Teilabschnitt des jeweiligen zweiten Längsabschnitts ein vorderes oder hinteres Windelseitenteil einer unmittelbar angrenzenden weiteren Inkontinenzwegwerfwindel bildet,
- wobei die gekrümmten Ränder der Längsabschnitte nach dem Abtrennen von der Windelseitenteilbahn derart konturiert sind, dass ein jedes hintere Windelseitenteil einen unteren Rand aufweist, der einen konvexen Abschnitt aufweist und ein jedes vordere Windelseitenteil einen oberen Rand aufweist, der einen konkaven Abschnitt aufweist, und dass die Länge E eines äußeren Randes eines jeweiligen vorderen Windelseitenteils kleiner ist als die Länge D eines inneren Randes eines jeweiligen vorderen Windelseitenteils,
- und wobei die Längserstreckung der vorderen Windelseitenteile mindestens 60% der Längserstreckung der hinteren Windelseitenteile beträgt.

Nach dem erfindungsgemäßen Verfahren wird also eine Windelhauptteilbahn endlos gefördert, und an diese Windelhauptteilbahn werden in der beanspruchten Weise Windelseitenteile in der Herstellungsmaschine angefügt. Die Windelseitenteile stammen ihrerseits von einer ggf. auch von mehreren endlos zugeführten Windelseitenteilbahnen, von der doppelnutzige Längsabschnitte abgetrennt und dann auf die endlos geförderte Windelhauptteilbahn appliziert und an dieser fixiert werden. Es wird zum Vereinzeln der Inkontinenzwegwerfwindeln ein Trennschnitt durch einen jeweiligen Längsabschnitt hindurchgeführt, so dass der eine Teilabschnitt dieses Längsabschnitts zu dem einen Inkontinenzartikel und der andere Teilabschnitt zum angrenzenden Inkontinenzartikel gehört. Ein wesentlicher Vorteil dieses Verfahrens ist, dass die vorderen und hinteren Windelseitenteile bereits durch den Trennvorgang, also durch die Konturierung des Trennschnitts beim Abtrennen der doppelnutzigen Längsabschnitte, ihre näher spezifizierte konkave bzw. konvexe Kontur erhalten und somit nach dem Anfügen der Längsabschnitte an die Hauptteilbahn kein weiteres die Beinöffnung konturierendes Schneiden der Abschnitte erforderlich ist.

Vorzugsweise sind die gekrümmten Ränder der Längsabschnitte nach dem Abtrennen von der Windelseitenteilbahn derart konturiert, dass ein jedes hintere Windelseitenteil einen unteren Rand aufweist, der einen einzigen konvexen Abschnitt aufweist und/oder ein jedes vordere Windelseitenteil einen unteren Rand aufweist, der einen einzigen konkaven Abschnitt aufweist. Darüber hinaus ist es vorteilhaft, wenn solchenfalls die Quererstreckung des konvexen Abschnitts sich über die gesamte Breite des unteren Randes des hinteren Seitenteils erstreckt und/oder die Quererstreckung des konkaven Abschnitts sich über die gesamte Breite des oberen Randes des vorderen Seitenteils erstreckt.

Der Windelhauptteil bzw. die Windelhauptteilbahn können prinzipiell in der Längsrichtung kontinuierlich insbesondere aus Vlies- oder Folienmaterial oder aus einem Vlies/FolienVerbund hergestellt werden. Es erweist sich aber auch als vorteilhaft, wenn ein jeweiliger Windelhauptteil einen Flüssigkeiten speichernden, vorzugsweise superabsorbierende Materialien umfassenden Absorptionskern aufweist, der insbesondere als vorgefertigte Einheit auf eine Trägerbahn der Windelhauptteilbahn aufgebracht werden kann. Beispielsweise könnte diese Trägerbahn eine Folienschicht umfassen oder auch eine Verbundfolie mit einer dünnen Vliesstoffauflage auf der späteren Außenseite, wobei dann auf diese Trägerbahn in Längsrichtung aufeinander folgend und in einem Abstand zueinander Absorptionskerne aufgebracht und vorzugsweise gegen die Trägerbahn fixiert werden. Dies kann wie erwähnt ein vorkonfektionierter Absorptionskern sein, häufig auch als Saugkörper bezeichnet, oder der Absorptionskern kann durch Ablage von absorbierendem Fasermaterial vorzugsweise mit superabsorbierenden Polymermaterialien konstruktiv gebildet sein.

Das Abtrennen der Längsabschnitte von der Windelseitenteilbahn erfolgt vorzugsweise als Schnitt oder Stanzung entlang von Trennlinien.

Nach einer Variante des Verfahrens nach Anspruch 1, bildet ein zweiter Teilabschnitt des jeweiligen ersten Längsabschnitts ein vorderes Windelseitenteil und ein zweiter Teilabschnitt des jeweiligen zweiten Längsabschnitts ein hinteres Windelseitenteil. Die Inkontinenzwindeln werden solchenfalls derart hergestellt, dass das Rückenteil einer ersten Inkontinenzwindel vor der Vereinzelung unmittelbar angrenzt an das Vorderteil einer darauf folgenden Inkontinenzwindel. Bevorzugt erfolgt das Bilden und Abtrennen dieser doppelnutzigen Längsabschnitte dann dadurch, dass die endlose Windelseitenteilbahn zunächst durch einen sich in Längsrichtung wellenförmig oder sinusoidal kontinuierlich durch das Material der Windelseitenteilbahn hindurch erstreckenden Schnitt entlang einer Trennlinie in zwei endlose Teilbahnen geteilt und anschließend von jeder Teilbahn die doppelnutzigen Längsabschnitte quer zu der Längsrichtung endgültig abgetrennt werden. Die ersten und zweiten doppelnutzigen Längsabschnitte sind solchenfalls vorzugsweise deckungsgleich ausgebildet. Denkbar und vorteilhaft wäre auch, dass das Abtrennen der doppelnutzigen Längsabschnitte durch Trennen entlang einer wellenförmig oder sinusoidal oder bogenförmig verlaufenden, sich ein oder beidseitig über die Längsränder der Windelseitenteilbahn hinaus erstreckenden Trennlinie erfolgt.

Nach einem weiter bevorzugten Erfindungsgedanken des Verfahrens besteht hingegen ein abgetrennter doppelnutziger Längsabschnitt aus zwei Teilabschnitten, die entweder hintere Windelseitenteile oder vordere Windelseitenteile bilden.

Solchenfalls bildet ein zweiter Teilabschnitt des jeweiligen ersten Längsabschnitts ebenfalls ein hinteres Windelseitenteil und ein zweiter Teilabschnitt des jeweiligen zweiten Längsabschnitts bildet ein vorderes Windelseitenteil. Solchenfall erfolgt das Abtrennen der doppelnutzigen Längsabschnitte vorzugsweise durch Trennen entlang einer wellenförmig oder sinusoidal oder bogenförmig verlaufenden, sich ein oder beidseitig über die Längsränder der Windelseitenteilbahn hinaus erstreckenden Trennlinie.

Des Weiteren erweist es sich als vorteilhaft, dass in der Längsrichtung erstreckte erste elastische Elemente an die Windelhauptteilbahn angefügt werden, und zwar auf beiden Seiten. Diese elastischen Elemente können auch einer gewissen Konturierung entlang der Beinöffnungen folgend vorgesehen werden. Sie können aber auch exakt in Längsrichtung verlaufen.

Des Weiteren können in der ersten Längsrichtung erstreckte zweite elastische Elemente, insbesondere in Form von so genannten und an sich zum Beispiel aus EP0263720A1 bekannten aufstehenden Cuff-Elementen, an die Windelhauptteilbahn angefügt werden. Diese vorzugsweise aufstehenden zweiten elastischen Elemente flankieren gewissermaßen ein Zentrum des Windelhauptteils oder des Absorptionskerns; sie können im Bereich der Saugkörperränder, innerhalb der Saugkörperränder oder außerhalb der Saugkörperränder vorgesehen werden. Sie bilden einen Seitenauslaufschutz des Inkontinenzartikels.

Nach einer vorteilhaften Variante des erfindungsgemäßen Verfahrens erfolgt das Zuführen der Längsabschnitte zu der Trenn- und Applikationsstation mit einer ersten Geschwindigkeit v1 und das Zuführen der endlosen Windelhauptteilbahn zu der Trenn- und Applikationsstation mit einer zweiten Geschwindigkeit v2, wobei die erste Geschwindigkeit v1 geringer ist als die zweite Geschwindigkeit v2. Solchenfalls werden die Längsabschnitte der Windelseitenteilbahn bei verhältnismäßig geringer Geschwindigkeit v1 abgetrennt und dann vorzugsweise auf die Geschwindigkeit v2 beschleunigt, so dass die Längsabschnitte vorzugsweise bei derselben Geschwindigkeit v2 auf die Windelhauptteilbahn abgelegt werden. Diese Beschleunigung kann beispielsweise durch eine insbesondere unterdruckbeaufschlagbare Applikationswalze erfolgen, die in Transportrichtung der Windelseitenteilbahn einer Trenn- oder Schneidstation, wo die Längsabschnitte abgetrennt werden, insbesondere unmittelbar nachfolgend angeordnet ist.

Die zweite Geschwindigkeit v2 kann nach einer bevorzugten Ausführungsform der Erfindung um mindestens 40%, insbesondere um mindestens 70%, insbesondere um mindestens 90%, insbesondere um höchstens 200% größer sein als die erste Geschwindigkeit v1.

In vorteilhafter Weiterbildung des Verfahrens wird vorgeschlagen, dass die ersten Längsabschnitte nach dem Abtrennen von der Windelseitenteilbahn derart gekrümmte Ränder aufweisen und die Längsabschnitte derart an der Windelhauptteilbahn fixiert werden, dass nach dem Vereinzeln der Inkontinenzwegwerfwindeln, der untere Rand der hinteren Windelseitenteile eine Länge A und eine Breite B aufweist und der konvexe Abschnitt einen Extrempunkt P mit einer Längserstreckung E und einer Quererstreckung F aufweist, wobei das Verhältnis der Länge A zu der Breite B des unteren Randes eines hinteren Windelseitenteils 0,40-0,90 beträgt und das Verhältnis der Längserstreckung E des Extrempunktes P zu dessen Quererstreckung F 0,15-0,80 beträgt.

Im Rahmen der vorliegenden Erfindung wird hinsichtlich der Definition des Extrempunkts P, dessen Längserstreckung E und Quererstreckung F, des konvexen Abschnitts und der Länge A und der Breite B des unteren Randes der hinteren Windelseitenteile auf die DE102010026643 verwiesen.

Bevorzugt beträgt das Verhältnis der Länge A des unteren Randes eines hinteren Windelseitenteils zu der Breite B des unteren Randes eines hinteren Windelseitenteils 0,50-0,80, insbesondere 0,55-0,75.

In Weiterbildung der Erfindung wird vorgeschlagen, dass das Verhältnis der Quererstreckung F des Extrempunktes P zu der Breite B eines jeweiligen unteren Randes der hinteren Windelseitenteils 0,20-0,60, insbesondere 0,30-0,50 beträgt.

Weiter bevorzugt beträgt das Verhältnis der Längserstreckung E des Extrempunktes P zur der Länge A eines unteren Randes eines jeweiligen hinteren Windelseitenteils 0,10-0,40, insbesondere 0,10-0,30.

Vorteilhaft beträgt das Verhältnis der Längserstreckung E eines Extrempunktes P des konvexen Abschnittes eines hinteren Windelseitenteils zu dessen Quererstreckung F 0,20-0,60 insbesondere 0,20-0,50.

Das Flächengewicht des die Windelseitenteilbahn und damit die Windelseitenteile bildenden Materials sollte vorzugsweise 13-40 g/m², insbesondere 15-30 g/m² und ganz besonders 16-28 g/m² betragen.

Es hat sich weiter als vorteilhaft erwiesen, dass die vorderen und/oder hinteren Windelseitenteile, mithin die Windelseitenteilbahn, aus einem Vliesstoffmaterial bestehen oder ein Vliesmaterial umfassen. Geeignet sind insbesondere alle Vliesstoffmaterialien, die zumindest eine Rezepturkomponente auf Basis eines thermoplastischen Polymers enthalten. Die Vliesstoffe können Fasern aus PE, PP, PET, Rayon, Cellulose, PA und Mischungen dieser Fasern enthalten. Auch Bi- oder Multikomponentenfasern sind denkbar und vorteilhaft. Vorteilhaft sind insbesondere Kardenvliese, wasserstrahlvernadelte Vliese, Spunbond(S)-Vliese, Meltblown(M)-Vliese, SMS-Vliese, SMMS-Vliese oder auch Laminate aus einer oder mehreren dieser Vliesarten, wobei S für Spunbond- und M für Meltblown-Vliesschichten steht. Denkbar und vorteilhaft ist des Weiteren, die vorderen und/oder hinteren Windelseitenteile aus einem Vliesstoff-Folienlaminat zu bilden. Solchenfalls würde die Folienkomponente außen zu liegen kommen und die Vliesstoffkomponente innen, um körperzugewandt eine weiche Oberfläche zu gewährleisten. In Weiterbildung dieses Erfindungsgedankens ist vorteilhaft, die vorderen und/oder hinteren Windelseitenteile aus einem Vliesstoff-Folie-Vliesstofflaminat zu bilden, bei dem eine Folienkomponente sandwichartig zwischen zwei Vlieskomponenten angeordnet ist.

In Weiterbildung der Erfindung ist vorgesehen, dass die Windelseitenteilbahn vor dem Abtrennen der doppelnutzigen Längsabschnitte mit Verschlussmitteln, insbesondere mit mechanische Verschlusshilfen aufweisenden Verschlussmitteln versehen wird, und zwar im Bereich der späteren hinteren Windelseitenteile. Die Verschlussmittel sind zum bestimmungsgemäßen Festlegen der Inkontinenzwegwerfwindel an den Körper eines Menschen zumindest bereichsweise sowohl an der Außenseite des Windelhauptteils als auch an der Außenseite der vorderen Windelseitenteile lösbar festlegbar, wobei die Haltekräfte zwischen den Verschlussmitteln und der Außenseite der vorderen Windelseitenteile vorzugsweise größer sind als die Haltekräfte zwischen den Verschlussmitteln und der Außenseite des Windelhauptteils.

Die Außenseite des Windelhauptteils der Inkontinenzwegwerfwindel wird vorzugsweise zumindest bereichsweise, insbesondere aber vollflächig durch einen Vliesstoff gebildet. Dies vermittelt der Inkontinenzwegwerfwindel einen textilähnlichen Eindruck. Solchenfalls ist es vorteilhaft, das Backsheet des Windelhauptteils aus einem Vliesfolienlaminat zu bilden, wobei die Vlieslage außen und die Folienlage innen zum Saugkörper gerichtet zu liegen kommt, so dass die Vlieslage die Außenseite des Windelhauptteils bildet. Damit ist zum einen die Flüssigkeitsundurchlässigkeit des Windelhauptteils sichergestellt und zum anderen der hautfreundliche Charakter der Windel gesichert. Die Folienlage dieses Vliesfolienlaminates ist dann vorzugsweise aus einer ein- oder mehrschichtigen flüssigkeitsundurchlässigen, vorzugsweise aber gleichwohl atmungsaktiven Folie gebildet, wobei die Atmungsaktivität der vorderen und/oder der hinteren Windelseitenteile vorzugsweise größer ist als die Atmungsaktivität des das Backsheet der Inkontinenzwegwerfwindel bildenden Vliesfolienlaminates.

In Weiterbildung der Erfindung erweist es sich als vorteilhaft, die doppelnutzigen Längsabschnitte beim Abtrennen von der Windelseitenteilbahn derart zu dimensionieren, und derart an die Hauptteilbahn anzufügen, dass die Länge der späteren vorderen und/oder hinteren Windelseitenteile, also deren maximale Erstreckung in Windellängsrichtung mindestens 15 cm, insbesondere mindestens 20 cm, weiter insbesondere mindestens 25 cm, weiter insbesondere mindestens 27 cm und weiter insbesondere höchstens 45 cm beträgt. Vorzugsweise weisen die hinteren und/oder die vorderen Windelseitenteile an ihrem jeweiligen inneren Rand ihre maximale Erstreckung in der Windellängsrichtung auf.
In besonders vorteilhafter Weise nimmt die Länge eines jeden vorderen Windelseitenteils ausgehend von dem Seitenrand des Hauptteils nach außen hin (also in Querrichtung) ab. In Längsrichtung betrachtet verschmälern sich somit die Windelseitenteile vorzugsweise nach außen hin, so dass ein äußerer Rand der vorderen Windelseitenteile eine geringere Länge aufweist als ein innerer Rand der vorderen Windelseitenteile.

Vorteilhafterweise beträgt die Gesamtlänge der Inkontinenzwegwerfwindel 60-120 cm, insbesondere 65-115 cm und weiter insbesondere 70-110 cm. In Weiterbildung der Erfindung erweist es sich als vorteilhaft, wenn die doppelnutzigen Längsabschnitte beim Abtrennen von der Windelseitenteilbahn derart dimensioniert werden, dass die Breite der vorderen und/oder hinteren Windelseitenteile, also deren maximale Erstreckung über den Seitenrand des Windelhauptteils hinaus 10-40 cm, insbesondere 12-35 cm, weiter insbesondere 14-30 cm und weiter insbesondere höchstens 25 cm beträgt. Vorzugsweise weisen die vorderen Windelseitenteile die gleiche Breite auf wie die hinteren Windelseitenteile .

Es hat sich als vorteilhaft erwiesen, wenn die Länge der hinteren Windelseitenteile mindestens 10 %, insbesondere mindestens 15%, weiter insbesondere mindestens 20% und weiter insbesondere mindestens 22%, weiter insbesondere höchstens 40 % der Gesamtlänge der Inkontinenzwegwerfwindel beträgt.

Darüber hinaus sieht eine bevorzugte Ausführungsform vor, dass die vorderen Windelseitenteile eine geringere, insbesondere eine um mindestens 5%, weiter insbesondere eine um mindestens 10%, weiter insbesondere eine um mindestens 15% und weiter insbesondere eine um höchstens 30% geringere Längserstreckung aufweisen als die hinteren Windelseitenteile.

Vor- und nachstehend werden die Begriffe "Länge" und "Längserstreckung" synonym verwendet, sind also bedeutungsgleich.

In der Zeichnung zeigen
Figur 1 eine nicht maßstabsgerechte Draufsicht auf eine nach dem erfindungsgemäßen Verfahren hergestellte Inkontinenzwegwerfwindel in schematischer Darstellung mit beidseits angefügten Windelseitenteilen;
Figuren 2 eine schematische Darstellung des erfindungsgemäßen Herstellungsverfahrens
Figur 3 und 4 eine Draufsicht auf eine Windelseitenteilbahn in schematischer Darstellung mit angedeuteten Varianten der Erstreckung von Trennlinien zur Abtrennung von doppelnutzigen Längsabschnitten
Figur 5 eine Draufsicht auf eine Windelseitenteilbahn in schematischer Darstellung mit einer weiteren angedeuteten Variante der Erstreckung von Trennlinien zur Abtrennung von doppelnutzigen Längsabschnitten
Figur 6 eine Draufsicht auf eine Windelhauptteilbahn mit applizierten doppelnutzigen Längsabschnitten einer Windelseitenteilbahn
Figur 7 eine Draufsicht auf eine Windelseitenteilbahn in schematischer Darstellung mit einer weiteren angedeuteten Variante der Erstreckung von Trennlinien zur Abtrennung von doppelnutzigen Längsabschnitten
Figur 8 eine Draufsicht auf eine Windelseitenteilbahn in schematischer Darstellung mit einer weiteren angedeuteten Variante der Erstreckung von Trennlinien zur Abtrennung von doppelnutzigen Längsabschnitten
Figur 9 eine Draufsicht auf eine Windelhauptteilbahn mit applizierten doppelnutzigen Längsabschnitten einer Windelseitenteilbahn nach Figur 8

Figur 1 zeigt schematisch, nicht maßstabsgerecht eine Draufsicht auf die Innenseite, also die körperzugewandte Seite einer erfindungsgemäß hergestellten absorbierenden Inkontinenzwegwerfwindel 2 des offenen Typs (nachfolgend auch gelegentlich als Windel bezeichnet) in eben ausgefaltetem Zustand. Die Inkontinenzwegwerfwindel 2 umfasst einen Windelhauptteil 4 mit einem Vorderbereich 6, einem Rückenbereich 8 und einem in Längsrichtung 28 dazwischen liegenden Schrittbereich 10. Außerdem angedeutet ist ein Absorptionskern 12, der üblicherweise zwischen chassisbildenden Materialien des Windelhauptteils 4, also insbesondere zwischen einem flüssigkeitsdurchlässigen aus einem Vliesstoffmaterial gebildeten Topsheet 11 und einem im Wesentlichen flüssigkeitsundurchlässigen aus einem Folienmaterial gebildeten Backsheet 13 des Windelhauptteils 4 angeordnet ist. Das Backsheet 13 kann auch aus einem flüssigkeitsundurchlässigem Vliesstoff oder einem Vliesfolienlaminat gebildet sein, wobei die Vlieslage dann außen und die Folienlage innen zum Absorptionskern gerichtet zu liegen kommt. Dies vermittelt der Inkontinenzwegwerfwindel 2 einen textilähnlichen Eindruck. Seitlich neben den Längsrändern des Absorptionskerns 12 sind erste elastische Elemente 80 an den Windelhauptteil 4, zwischen Topsheet 11 und Backsheet 13 angefügt. Die elastischen Elemente 80 verlaufen im Wesentlichen in der Längsrichtung 28, also mit einer wesentlichen Komponente in Längsrichtung, wobei sie im dargestellten Fall einen gekrümmten Verlauf entlang des dem Schrittbereich 10 zuzuordnenden Beinöffnungsbereichsabschnittes nehmen.

Die Inkontinenzwegwerfwindel 2 umfasst des Weiteren vordere Windelseitenteile 22 und hintere Windelseitenteile 20, die als vier separate Vliesstoffkomponenten beidseits an den Windelhauptteil 4 angefügt sind. Der untere Rand 65 der hinteren Windelseitenteile 20 weist einen konvexen Abschnitt 44 auf, während der obere Rand 66 der vorderen Windelseitenteile 22 eine konkaven Abschnitt 45 aufweist. In der dargestellten Ausführungsform ist erkennbar, dass die gekrümmten Ränder der Längsabschnitte nach dem Abtrennen von der Windelseitenteilbahn derart konturiert sind, dass ein jedes hintere Windelseitenteil 20 einen unteren Rand 65 aufweist, der einen einzigen konvexen Abschnitt 44 aufweist und ein jedes vordere Windelseitenteil 22 einen oberen Rand 66 aufweist, der einen einzigen konkaven Abschnitt 45 aufweist. Darüber hinaus erstreckt sich der konvex Abschnitt 44 in Querrichtung 30 über die gesamte Breite B des unteren Randes 65 eines hinteren Seitenteils 20 und der konkave Abschnitt 45 erstreckt sich in Querrichtung 30 über die gesamte Breite C des oberen Randes 66 eines vorderen Seitenteils 22. Außerdem ist erkennbar, dass ein äußerer Rand 67 der vorderen Windelseitenteile 22 eine geringere Länge E aufweist als ein jeweiliger innerer Rand 68 mit der Länge D. Die Längserstreckung der vorderen Windelseitenteile 22 beträgt im dargestellten Fall 65 % der Längserstreckung der hinteren Windelseitenteile 20. Damit ist beim Anlegen der Windel an den Körper eines Menschen ausreichend Spielraum zum Fixieren der Verschlussmittel gegeben.

Im dargestellten Fall entspricht die Länge D des inneren Randes 68 der vorderen Windelseitenteile 22 der maximalen Erstreckung der vorderen Windelseitenteile 22 in der Längsrichtung 28, mithin der Länge der vorderen Windelseitenteile 22. Auch die Länge des inneren Randes 69 der hinteren Windelseitenteile 20 entspricht der maximalen Erstreckung der hinteren Windelseitenteile 20 in der Längsrichtung 28, mithin der Länge der hinteren Windelseitenteile 22. Die Konturierung der der Beinöffnung zugewandten Ränder 65, 66 der hinteren und vorderen Windelseitenteile 20, 22 könnte jedoch auch derart ausgebildet sein, dass die maximale Erstreckung der Windelseitenteile sich in Querrichtung betrachtet außerhalb der inneren Ränder 69, 68 der hinteren und vorderen Windelseitenteile 20, 22 befindet.

Die Windelseitenteile 20, 22 sind in einem Überlappungsbereich mit chassisbildenden Materialien des Windelhauptteils 4, also beispielsweise mit dem Backsheet 13 und/oder dem Topsheet 11 unlösbar verbunden. Die Windelseitenteile 20, 22 erstrecken sich über die vorderen und hinteren seitlichen Längsränder 42, 41 des Windelhauptteils in Querrichtung 30 hinaus. Im Rahmen der vorliegenden Erfindung wird das Verständnis zugrunde gelegt, dass der jeweilige innere Rand 69, 68 der Windelseitenteile 22, 20 durch den Übergang zum Windelhauptteil 4, mithin durch den hinteren 41 bzw. vorderen Längsrand 42 Hauptteils 4 gebildet wird.

Unter vorderen und hinteren seitlichen Längsrändern 42, 41 des Windelhauptteils werden im Rahmen der vorliegenden Erfindung somit diejenigen Längsrandbereiche des Windelhauptteils verstanden, an die die Windelseitenteile angefügt sind und über welche diese sich hinaus erstrecken. Die Längserstreckung der vorderen und hinteren Seitenränder des Windelhauptteils 42, 41 definieren damit auch die Längserstreckung des Vorderbereiches 6 und des Rückenbereiches 8 der Inkontinenzwegwerfwindel 2.

Die Windelseitenteile 20, 22 sind dafür gedacht und bestimmt, im angelegten Zustand der Inkontinenzwegwerfwindel 2 miteinander verbunden zu werden, um einen in Umfangsrichtung durchgehenden Hüftbereich der Windel zu bilden. Dabei werden jeweils die auf einer Seite des Windelhauptteils 4 vorgesehenen Windelseitenteile 20, 22 miteinander verbunden. Hierzu sind an den hinteren Windelseitenteilen 20 Verschlussmittel 33, insbesondere mit mechanischen Verschlusshilfen wie Kletthaken, vorgesehen, welche auf der Außenseite der vorderen Windelseitenteile 22 lösbar festlegbar sind. Vorzugsweise sind die Verschlussmittel außerdem auf der Außenseite des Windelhauptteils 4 und weiter vorzugsweise auch auf der Außenseite der hinteren Windelseitenteile 20 lösbar festlegbar, so dass der Inkontinenzartikel sehr variabel an die anatomischen Gegebenheiten des Trägers angepasst werden kann. Sowohl die vorderen Windelseitenteile 22 als auch die hinteren Windelseitenteile 20 sind aus einem Vliesstoffmaterial, im dargestellten Fall aus einem PP-Spinnvlies, Pegatex S, Hersteller: Pegas a.s.,Primetická 86, 66904 Znojmo, CZ, gebildet. Das Flächengewicht des Vliesstoffmaterials der vorderen und hinteren Windelseitenteile beträgt 27 g/m². Die Faserstärke der das Vliesstoffmaterial bildenden Fasern beträgt 2 dtex.

Wie aus Figur 1 erkennbar ist, weisen die hinteren Windelseitenteile 20 eine größere Flächenerstreckung auf als die vorderen Windelseitenteile 22.

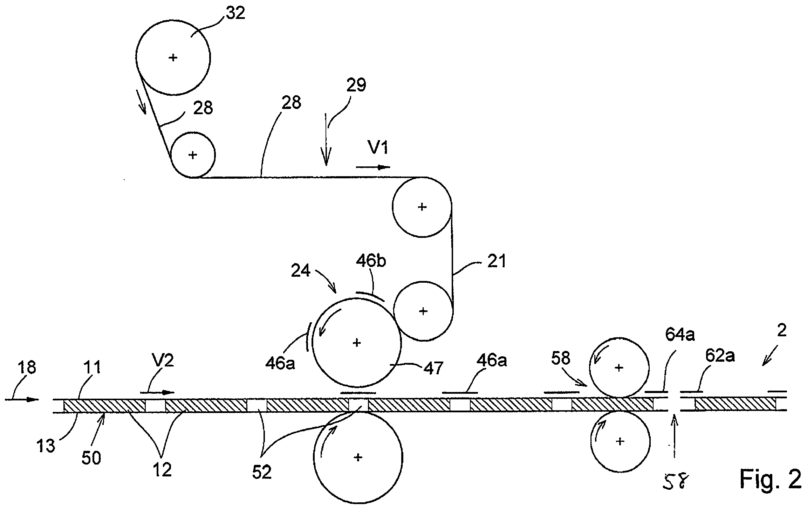

Figur 2 verdeutlicht schematisch ein erfindungsgemäßes Verfahren zum Herstellen einer Vielzahl von erfindungsgemäßen Inkontinenzwegwerfwindeln.

Zur Herstellung der Windel 2 wird gemäß Figur 2 (oberer Bereich) eine Windelseitenteilbahn 21 gebildet und in Richtung auf eine Trenn- und Applikationsstation 24 gefördert. Die Windelseitenteilbahn 21 umfasst eine von einer Vorratsrolle 32 endlos abrollbare Bahn 28 aus einem im Wesentlichen insbesondere undehnbaren Vliesstoffmaterial. An einer nur schematisch angedeuteten Tapestation 29 werden an Längsrändern der Bahn getaktet Verschlusstapes angefügt, welche die späteren Verschlussmittel 33 der Windel 2 bilden (Figur 1). Die Bahn 28 wird weiter mit der Geschwindigkeit v1 in Richtung auf eine insgesamt mit dem Bezugszeichen 24 versehene Trenn- und Applikationsstation gefördert, wo doppelnutzige (das heißt später je zwei Teilabschnitte bzw. je zwei Windelseitenteile bildende) aufeinander folgende erste und zweite Längsabschnitte 46a, 46b von der Windelseitenteilbahn 21 abgetrennt und über eine schematisch angedeutete Beschleunigungswalze 47 auf eine Geschwindigkeit v2 beschleunigt und der Windelhauptteilbahn 50 zugeführt werden.

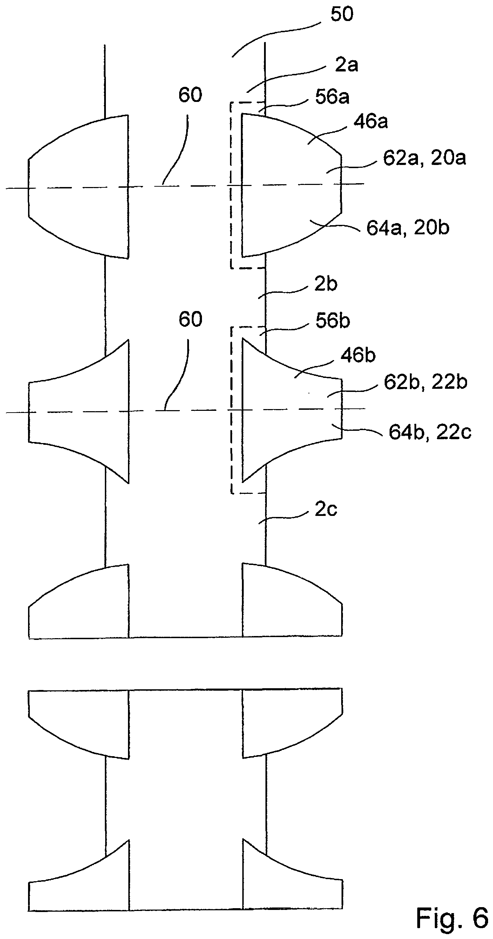

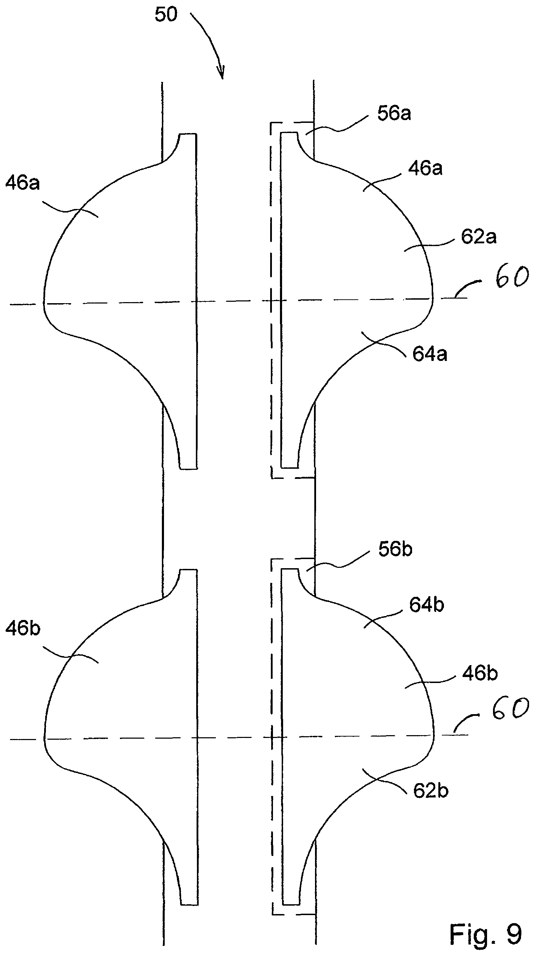

In Figur 2 unten ist das Zuführen und Fördern einer endlosen Windelhauptteilbahn 50 dargestellt. Die Windelhauptteilbahn 50 trägt eine endlose Anzahl von aufeinander folgend abgelegten und voneinander durch Zwischenräume 52 beabstandeten Absorptionskernen 12. Des Weiteren angedeutet ist eine Topsheetlage 11 und eine Backsheetlage 13. Absorptionskern 12 und Topsheetlage 11 werden im Folgenden als zu der Windelhauptteilbahn 50 gehörig angesehen. Die Windelhauptteilbahn wird in Richtung auf die Trenn- und Applikationsstation 24 mit der Geschwindigkeit v2 endlos gefördert. In der Trenn- und Applikationsstation 24 werden die Längsabschnitte 46a, 46b der Windelseitenteilbahn 22, wie aus Figur 2 ersichtlich, auf die Windelhauptteilbahn 50 appliziert und daran angefügt, und zwar derart, dass sie an den Seitenrändern der Hauptteilbahn 50 die späteren Trennlinien 60 (siehe Figur 6), entlang derer die Windeln in einem nachgeordneten Verfahrensschritt vereinzelt werden, überfangen oder in diesem Bereich angeordnet werden. Dies ist beispielsweise auch in der Draufsicht aus den Figuren 6 und 9 ersichtlich. In Figuren 6 und 9 sind diese Längsrandbereiche der Hauptteilbahn 50 mit dem Bezugszeichen 56a oder 56b bezeichnet. Der so erhaltene Verbund wird weiter in Richtung auf eine Vereinzelungsstation 58 gefördert, wo im Wesentlichen quer zur Förderrichtung, die der Längsrichtung 18 der herzustellenden Windeln 2 entspricht, ein Trennschnitt ausgeführt wird, beispielsweise mit einer rotierenden Messerwalze oder mit einem Stanzwerkzeug. Der Trennschnitt oder die Trennlinie ist in den Figuren 6 und 9 mit dem Bezugszeichen 60 angedeutet. Er wird so ausgeführt, dass er jeweils durch einen applizierten Längsabschnitt 46a, 46b verläuft und diesen in einen jeweiligen ersten Teilabschnitt 62a, 62b und in einen jeweiligen zweiten Teilabschnitt 64a, 64b teilt, welche die Windelseitenteile bilden (siehe auch Figuren 6 und 9).

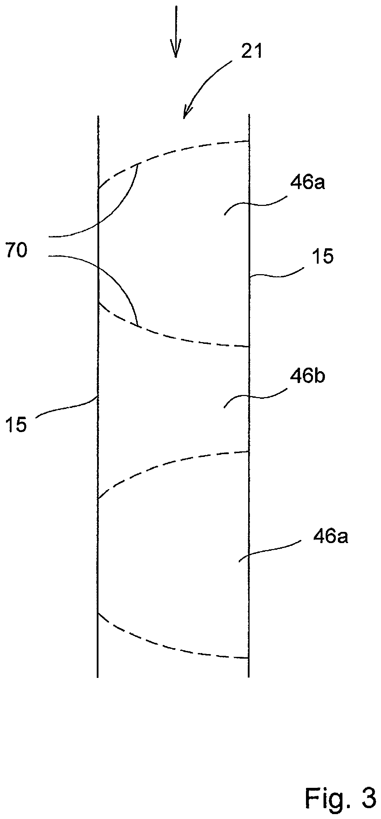

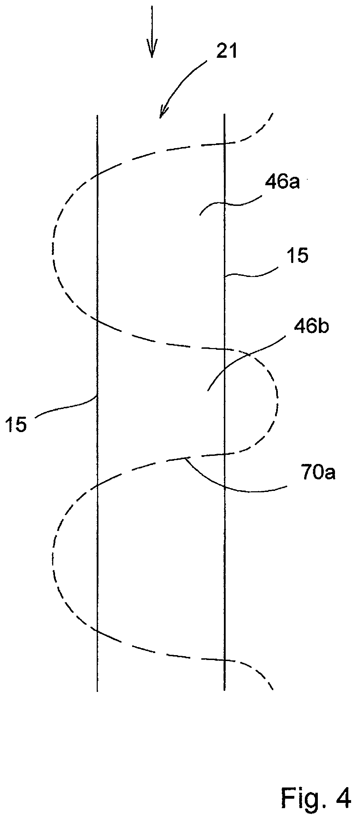

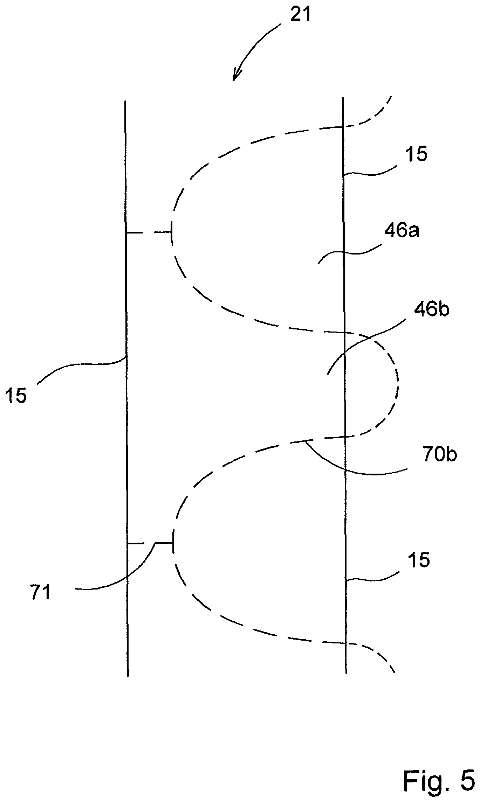

Verschiedene erfindungsgemäße Verfahrensvarianten werden nun anhand der Figuren 3 bis 9 beschrieben:
Nach der in Figur 3 dargestellten Verfahrensvariante wird eine Windelseitenteilbahn 21 in Pfeilrichtung gefördert. Man erkennt gestrichelt dargestellt die bogenförmigen Trennlinien 70 entlang derer die ersten und zweiten doppelnutzigen Längsabschnitte 46a, 46b abgetrennt werden. Es wäre auch denkbar und vorteilhaft, das Abtrennen der Längsabschnitte entlang einer Trennlinie 70a zu vollziehen, die sich wellenförmig oder sinusoidal beidseitig über die Längsränder 15 der Windelseitenteilbahn 21 hinauserstreckt (Figur 4). In einer alternativen Variante des Verfahrens (Figur 5) erfolgt das Abtrennen entlang einer Trennlinie 70b, die sich wellenförmig oder sinusoidal einseitig, das heißt nur über einen der Längsränder 15 der Windelseitenteilbahn 21 hinauserstreckt, so dass die endgültige Abtrennung der zweiten Längsabschnitte 46b einen weiteren Schnitt oder einer weitere Stanzung erfordert, die/der dann im Wesentlichen quer zur Längsrichtung entlang einer weiteren Trennlinie 71 erfolgt. Dieser weitere Trennvorgang kann zeitlich versetzt oder im Wesentlichen gleichzeitig mit dem ersten Trennvorgang an einer entsprechend konfigurierten Trennstation erfolgen.

Figur 6 zeigt schematisch eine Windelhauptteilbahn 50, auf die beidseits der Bahn 50 voneinander beabstandete erste Längsabschnitte 46a und zweite Längsabschnitte 46b einer Windelseitenteilbahn 22 aufgebracht und beispielsweise durch Ultraschallschweißen, durch Kleber oder in sonstiger Weise unlösbar mit der Windelhauptteilbahn 50 verbunden worden sind. In Figur 6 oben ist ein Windelvereinzelungstrennschnitt oder eine Trennlinie 60 zum Vereinzeln der Windeln 2 lediglich angedeutet, während in Figur 6 unten die Vereinzelungsstation 58 (Figur 2) bereits durchlaufen wurde und der Windelvereinzelungstrennschnitt 60 ausgeführt wurde. Dabei wird jeweils ein erster Teilabschnitt 62a, 62b und ein zweiter Teilabschnitt 64a, 64b gebildet, wobei vorliegend ein erster Teilabschnitt 62a eines ersten Längsabschnitts 46a ein hinteres Windelseitenteil 20a einer ersten Windel 2a bildet, ein zweiter Teilabschnitt 64 a eines ersten Längsabschnitts 46a ein hinteres Windelseitenteil 20b einer darauf folgenden zweiten Windel 2b bildet, ein erster Teilabschnitt 62b eines zweiten Längsabschnitts 46b ein vorderes Windelseitenteil 22a der zweiten Windel 2b bildet, und ein zweiter Teilabschnitt 64b eines zweiten Längsabschnitts 46b ein vorderes Windelseitenteil 22c einer darauf folgenden dritten Windel 2c bildet.

Sämtliche für die Herstellung der Windel 2 benötigten Windelseitenteile beidseits des Windelhauptteils können durch eine einzige Windelseitenteilbahn bereitgestellt werden. An der in Figur 2 schematisch dargestellten Trenn- und Applikationsstation werden die abgetrennten Längsabschnitte erforderlichenfalls gedreht oder umgekehrt und an den jeweiligen Längsrändern des Windelhauptteils positioniert. Denkbar und vorteilhaft wäre hingegen auch, der Windelhauptteilbahn zwei der beschriebenen Windelseitenteilbahnen zuzuführen, um den Aufwand des Drehens und/oder Umkehrens der Längsabschnitte und/oder des Positionierens an beide Längsränder der Hauptteilbahn zu minimieren bzw. ganz zu vermeiden.

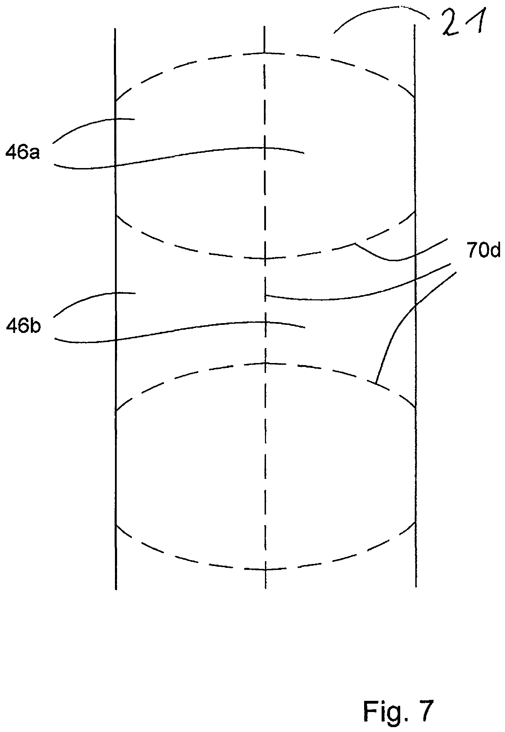

Nach einer weiteren Variante, die Figur 7 zeigt, werden von einer einzigen Windelseitenteilbahn 21 in Querrichtung betrachtet jeweils zwei doppelnutzige nebeneinander angeordnete erste und zweite Längsabschnitte 46a, 46b abgetrennt. Die erforderlichen Trennschnitte sind durch gestrichelte Linien 70d angedeutet.

Bei den in Figuren 3-7 dargestellten Varianten des erfindungsgemäßen Verfahrens besteht ein doppelnutziger Längsabschnitt aus zwei Teilabschnitten die entweder hintere Windelseitenteile oder vordere Windelseitenteile bilden.

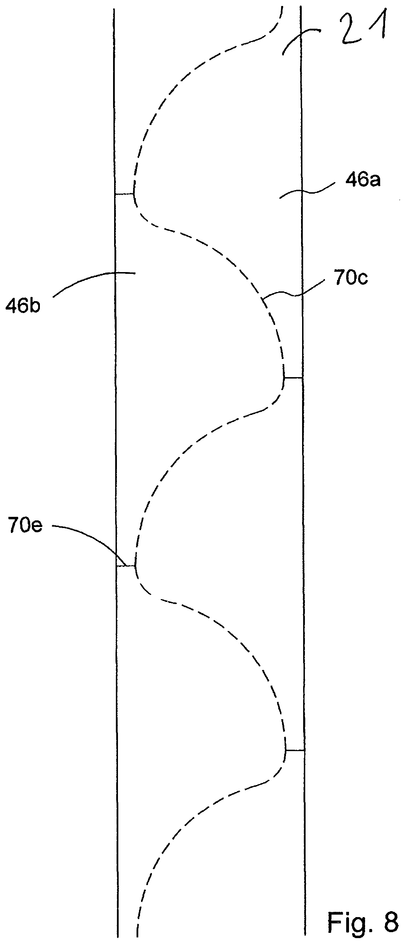

In der nachfolgend beschriebenen weiteren Variante umfasst ein doppelnutziger Längsabschnitt jeweils sowohl einen Teilabschnitt, der ein hinteres Windelseitenteil bildet als auch einen Teilabschnitt, der ein vorderes Windelseitenteil bildet: In Figur 8 ist erkennbar, dass eine Trennlinie 70c sich sinusoidal oder wellenförmig kontinuierlich mit einer Komponente in Längsrichtung durch die Windelseitenteilbahn 21 hindurcherstreckt. Die Bahn 21 könnte dann entlang dieser Trennlinie 70c zunächst in zwei Teilbahnen geteilt werden, und erst anschließend würden mittels weiterer quer orientierter Trennschnitte 70e die doppelnutzigen Längsabschnitte abgetrennt werden. Die Trennvorgänge könnten aber an einer einzigen Trenn- und Applikationsstation auch im Wesentlichen zeitgleich erfolgen.

Figur 9 zeigt schematisch eine Windelhauptteilbahn 50, auf die beidseits der Bahn 50 voneinander beabstandete erste Längsabschnitte 46a und zweite Längsabschnitte 46b der in Figur 8 dargestellten Windelseitenteilbahn 21 aufgebracht und beispielsweise durch Ultraschallschweißen, durch Kleber oder in sonstiger Weise unlösbar mit der Windelhauptteilbahn 50 an Bereichen 56a, 56b des Längsrandes der Bahn verbunden worden sind. In Figur 9 ist ein jeweiliger Windelvereinzelungstrennschnitt oder eine Trennlinie 60 zum Vereinzeln der Windeln 2 lediglich angedeutet. Es ist erkennbar, dass die zu vereinzelnden Windeln an der noch endlosen Bahn jeweils mit ihrem Rückenbereich an den Vorderbereich der darauffolgenden Windel angrenzen. Ein jeweiliger Längsabschnitt 46a, 46b wird mit dem Windelvereinzelungsschnitt daher derart geteilt, dass aus jedem Längsabschnitt 46a, 46b je ein ein vorderes und ein hinteres Windelseitenteil bildender Teilabschnitt 62a, 64a, 62b, 64b resultieren.

Mit der vorliegenden Erfindung ist es somit gelungen, ein kostengünstiges und prozesstechnisch vorteilhaft realisierbares Verfahren zum Herstellen einer Inkontinenzwegwerfwindel für Erwachsene mit je zwei separat angefügten hinteren Windelseitenteilen und vorderen Windelseitenteilen zu schaffen, wobei die hinteren Windelseitenteile einen konvexen Abschnitt und die vorderen Windelseitenteilen einen konkaven Abschnitt aufweisen.

## Patentansprüche

1. Verfahren zum Herstellen einer Vielzahl von einen Windelhauptteil (4) und daran angefügte vordere und hintere Windelseitenteile (22,20) aufweisenden Inkontinenzwegwerfwindeln (2), **gekennzeichnet durch** folgende Verfahrensschritte:
- Zuführen und Fördern einer endlosen Windelhauptteilbahn (50) in einer ersten Längsrichtung (18) zu einer Trenn- und Applikationsstation (24),
- Zuführen einer endlosen Windelseitenteilbahn (21) zu der Trenn- und Applikationsstation (24), wobei an der Trenn- und Applikationsstation (24) aufeinander folgende erste und zweite doppelnutzige Längsabschnitten (46a, 46b) mit zumindest bereichsweise gekrümmten Rändern von der Windelseitenteilbahn (21) abgetrennt werden und an ersten und zweiten Bereichen (56a,56b) der Windelhauptteilbahn angefügt werden, wobei die ersten und zweiten Bereiche (56a,56b) jeweils einen Hüftbereich zweier in Längsrichtung aufeinander folgender und aneinander angrenzender Windelhauptteile (4) umfassen,
- Vereinzeln der Inkontinenzwegwerfwindeln (2) durch Trennen der Windelhauptteilbahn (50) quer zur ersten Längsrichtung (18), wobei das Trennen durch die Längsabschnitte (46a,46b) hindurchgeführt wird, derart, dass ein erster Teilabschnitt (62a) zumindest eines ersten jeweiligen Längsabschnitts (46a) ein hinteres Windelseitenteil (20) einer ersten Inkontinenzwegwerfwindel bildet und ein zweiter Teilabschnitt (64a) des jeweiligen ersten Längsabschnitts (46a) ein vorderes oder hinteres Windelseitenteil (22, 20) einer unmittelbar angrenzenden zweiten Inkontinenzwegwerfwindel bildet, und dass ein erster Teilabschnitt (62b) eines zweiten jeweiligen Längsabschnitts (46b) ein vorderes Windelseitenteil (22) einer Inkontinenzwegwerfwindel bildet und ein zweiter Teilabschnitt (64b) des jeweiligen zweiten Längsabschnitts (46b) ein vorderes oder hinteres Windelseitenteil (22, 20) einer unmittelbar angrenzenden weiteren Inkontinenzwegwerfwindel bildet,
- wobei die gekrümmten Ränder der Längsabschnitte (46a, 46b) nach dem Abtrennen von der Windelseitenteilbahn (21) derart konturiert sind, dass nach dem Fixieren der ersten und zweiten Längsabschnitte (46a,46b) an den ersten und zweiten Bereichen (56a,56b) der Windelhauptteilbahn ein jedes hintere Windelseitenteil (20) einen unteren Rand (65) aufweist, der einen konvexen Abschnitt aufweist und ein jedes vordere Windelseitenteil (22) einen oberen Rand (66) aufweist, der einen konkaven Abschnitt aufweist, und dass die Länge E eines äußeren Randes (67) eines jeweiligen vorderen Windelseitenteils (22) kleiner ist als die Länge D eines inneren Randes (68) eines jeweiligen vorderen Windelseitenteils (22),
- und wobei die Längserstreckung der vorderen Windelseitenteile (22) mindestens 60% der Längserstreckung der hinteren Windelseitenteile (20) beträgt.

2. Verfahren nach Anspruch 1, **dadurch gekennzeichnet, dass** ein zweiter Teilabschnitt (64a) des jeweiligen ersten Längsabschnitts (46a) ein hinteres Windelseitenteil (20) bildet und ein zweiter Teilabschnitt (64b) des jeweiligen zweiten Längsabschnitts (46b) ein vorderes Windelseitenteil (22) bildet.

3. Verfahren nach Anspruch 1, **dadurch gekennzeichnet, dass** ein zweiter Teilabschnitt (64a) des jeweiligen ersten Längsabschnitts (46a) ein vorderes Windelseitenteil (22) bildet und ein zweiter Teilabschnitt (64b) des jeweiligen zweiten Längsabschnitts (46b) ein hinteres Windelseitenteil (22) bildet.

4. Verfahren nach einem oder mehreren der vorstehenden Ansprüche, **dadurch gekennzeichnet, dass** das Abtrennen der Längsabschnitte (46a, 46b) von der Windelseitenteilbahn (21) entlang von Trennlinien (70, 70a, 70b, 70c, 70d, 70e, 71) erfolgt.

5. Verfahren nach Anspruch 4, **dadurch gekennzeichnet, dass** eine Trennlinie (70c) sich sinusoidal oder wellenförmig kontinuierlich durch das Material der Windelseitenteilbahn (21) hindurch erstreckt und die Windelseitenteilbahn (21) hierdurch in zwei Teilbahnen teilt.

6. Verfahren einem der Anspruch 4, **dadurch gekennzeichnet, dass** eine Trennlinie (70, 70a, 70b, 70d) sich sinusoidal oder wellenförmig oder bogenförmig ein- oder beidseitig über die Längsränder der Windelseitenteilbahn (21) hinaus erstreckt.

7. Verfahren nach einem oder mehreren der vorstehenden Ansprüche, **dadurch gekennzeichnet, dass** in der ersten Längsrichtung (18) erstreckte erste elastische Elemente (80) an die Windelhauptteilbahn (50) angefügt werden.

8. Verfahren nach einem oder mehreren der vorstehenden Ansprüche, **dadurch gekennzeichnet, dass** das Zuführen der Windelseitenteilbahn (21) zu der Trenn- und Applikationsstation (24) mit einer ersten Geschwindigkeit v1 und das Zuführen der endlosen Windelhauptteilbahn (50) zu der Trenn- und Applikationsstation (24) mit einer zweiten Geschwindigkeit v2 erfolgt, wobei die erste Geschwindigkeit v1 geringer ist als die zweite Geschwindigkeit v2.

9. Inkontinenzwegwerfwindel hergestellt nach einem Verfahren nach einem der Ansprüche 1-8, **dadurch gekennzeichnet, dass** die Länge der hinteren Windelseitenteile (20) mindestens 15 cm, insbesondere mindestens 20 cm, weiter insbesondere mindestens 25 cm, weiter insbesondere mindestens 27 cm und weiter insbesondere höchstens 45 cm beträgt.

10. Inkontinenzwegwerfwindel nach Anspruch 9, **dadurch gekennzeichnet, dass** die Länge der hinteren Windelseitenteile (20) mindestens 10 %, insbesondere mindestens 15%, weiter insbesondere mindestens 20% und weiter insbesondere mindestens 22%, weiter insbesondere höchstens 40 % der Gesamtlänge der Inkontinenzwegwerfwindel beträgt.

11. Inkontinenzwegwerfwindel nach Anspruch 9 oder 10, **dadurch gekennzeichnet, dass** die vorderen Windelseitenteile (22) eine geringere, insbesondere eine um mindestens 5%, weiter insbesondere eine um mindestens 10%, weiter insbesondere eine um mindestens 15% und weiter insbesondere eine um höchstens 30 % geringere Längserstreckung aufweisen als die hinteren Windelseitenteile (20).

12. Inkontinenzwegwerfwindel nach einem oder mehreren der Ansprüche 9-11 **dadurch gekennzeichnet, dass** die Breite der vorderen und/oder der hinteren Windelseitenteile (22, 20) 10-40 cm, insbesondere 12-35 cm, weiter insbesondere 14-30 cm und weiter insbesondere höchstens 25 cm beträgt.

13. Inkontinenzwegwerfwindel nach einem oder mehreren der Ansprüche 9-12 **dadurch gekennzeichnet, dass** die vorderen Windelseitenteile (22) die gleiche Breite aufweisen wie die hinteren Windelseitenteile (20).

14. Inkontinenzwegwerfwindel nach einem oder mehreren der Ansprüche 9-13 **dadurch gekennzeichnet dass** der untere Rand (65) der hinteren Windelseitenteile (20) eine Länge A und eine Breite B aufweist und der konvexen Abschnitt einen Extrempunkt P aufweist, wobei das Verhältnis der Länge A zu der Breite B des unteren Randes (65) eines hinteren Windelseitenteils (20) 0,40-0,90 beträgt und das Verhältnis der Längserstreckung E des Extrempunktes P des konvexen Abschnittes eines hinteren Windelseitenteils (20) zu dessen Quererstreckung F 0,15-0,80 beträgt.

15. Absorbierende Inkontinenzwegwerfwindel (2) nach Anspruch 14, **dadurch gekennzeichnet, dass** das Verhältnis der Quererstreckung F des Extrempunktes P des konvexen Abschnittes eines hinteren Windelseitenteils (20) zu der Breite B des unteren Randes (65) eines hinteren Windelseitenteils (20) 0,20-0,60, insbesondere 0,30-0,50 beträgt.

16. Absorbierende Inkontinenzwegwerfwindel (2) nach Anspruch 14 oder 15, **dadurch gekennzeichnet, dass** das Verhältnis der Längserstreckung E des Extrempunktes P des konvexen Abschnittes eines hinteren Windelseitenteils (20) zur der Länge A des unteren Randes (65) eines hinteren Windelseitenteils (20) 0,10-0,40, insbesondere 0,10-0,30 beträgt.

17. Absorbierende Inkontinenzwegwerfwindel (2) nach einem oder mehreren der Ansprüche 14-16, **dadurch gekennzeichnet, dass** das Verhältnis der Längserstreckung E eines Extrempunktes P des konvexen Abschnittes eines hinteren Windelseitenteils (20) zu dessen Quererstreckung F 0, 20-0,60 insbesondere 0,20-0,50 beträgt.

18. Absorbierende Inkontinenzwegwerfwindel (2) nach einem oder mehreren der Ansprüche 14-17, **dadurch gekennzeichnet, dass** das Verhältnis der Länge A des unteren Randes (64) eines hinteren Windelseitenteils (22) zu der Breite B des unteren Randes (64) eines hinteren Windelseitenteils (22) 0,50-0,80, insbesondere 0,55-0,75 beträgt.

19. Absorbierende Inkontinenzwegwerfwindel (2) nach einem oder mehreren der Ansprüche 9-18, **dadurch gekennzeichnet, dass** die hinteren Windelseitenteile (20) mindestens ein mit Verschlusshilfen, insbesondere mit mechanischen Verschlusshilfen versehenes Verschlussmittel (33) aufweisen.

20. Absorbierende Inkontinenzwegwerfwindel (2) nach einem oder mehreren der Ansprüche 9-19, **dadurch gekennzeichnet, dass** die vorderen und hinteren Windelseitenteile (22, 20) aus einem Vliesstoffmaterial gebildet sind oder ein Vliesstoffmaterial umfassen.

## Claims

1. Method for producing a multiplicity of disposable incontinence diapers (2) having a diaper main part (4) and front and rear diaper side parts (22, 20) attached thereto, **characterized by** the following method steps of:
- feeding and conveying an endless diaper main-part web (50) in a first longitudinal direction (18) to a separating and application station (24),
- feeding an endless diaper side-part web (21) to the separating and application station (24), wherein, at the separating and application station (24), first and second dual-use longitudinal portions (46a, 46b) following one another and having at least regionally curved edges are severed from the diaper side-part web (21) and are attached to first and second regions (56a, 56b) of the diaper main-part web, wherein the first and second regions (56a, 56b) each comprise a hip region of two diaper main parts (4) following one another in the longitudinal direction and adjacent to one another,
- individually separating the disposable incontinence diapers (2) by separating the diaper main-part web (50) transversely to the first longitudinal direction (18), wherein the separating is carried out through the longitudinal portions (46a, 46b) such that a first sub-portion (62a) of at least one first respective longitudinal portion (46a) forms a rear diaper side part (20) of a first disposable incontinence diaper and a second sub-portion (64a) of the respective first longitudinal portion (46a) forms a front or rear diaper side part (22, 20) of an immediately adjacent second disposable incontinence diaper, and such that a first sub-portion (62b) of a second respective longitudinal portion (46b) forms a front diaper side part (22) of a disposable incontinence diaper and a second sub-portion (64b) of the respective second longitudinal portion (46b) forms a front or rear diaper side part (22, 20) of an immediately adjacent further disposable incontinence diaper,
- wherein the curved edges of the longitudinal portions (46a, 46b) are contoured after being severed from the diaper side-part webs (21), such that, after the first and second longitudinal portions (46a, 46b) have been fixed to the first and second regions (56a, 56b) of the diaper main-part web, each rear diaper side part (20) has a lower edge (65) which has a convex portion, and each front diaper side part (22) has an upper edge (66) which has a concave portion, and such that the length E of an outer edge (67) of a respective front diaper side part (22) is shorter than the length D of an inner edge (68) of a respective front diaper side part (22),
- and wherein the longitudinal extent of the front diaper side parts (22) is at least 60% of the longitudinal extent of the rear diaper side parts (20) .

2. Method according to Claim 1, **characterized in that** a second sub-portion (64a) of the respective first longitudinal portion (46a) forms a rear diaper side part (20) and a second sub-portion (64b) of the respective second longitudinal portion (46b) forms a front diaper side part (22).

3. Method according to Claim 1, **characterized in that** a second sub-portion (64a) of the respective first longitudinal portion (46a) forms a front diaper side part (22) and a second sub-portion (64b) of the respective second longitudinal portion (46b) forms a rear diaper side part (22).

4. Method according to one or more of the preceding claims, **characterized in that** the severing of the longitudinal portions (46a, 46b) from the diaper side-part web (21) takes place along separating lines (70, 70a, 70b, 70c, 70d, 70e, 71).

5. Method according to Claim 4, **characterized in that** one separating line (70c) extends in a sinusoidal or wave-like manner through the material of the diaper side-part web (21) and thereby divides the diaper side-part web (21) into two part-webs.

6. Method according to Claim 4, **characterized in that** one separating line (70, 70a, 70b, 70d) extends in a sinusoidal or wave-like or arcuate manner on one or both sides beyond the longitudinal edges of the diaper side-part web (21).

7. Method according to one or more of the preceding claims, **characterized in that** first elastic elements (80) that are made to extend in the first longitudinal direction (18) are attached to the diaper main-part web (50).

8. Method according to one or more of the preceding claims, **characterized in that** the feeding of the diaper side-part web (21) to the separating and application station (24) takes place at a first speed v1 and the feeding of the endless diaper main-part web (50) to the separating and application station (24) takes place at a second speed v2, wherein the first speed v1 is lower than the second speed v2.

9. Disposable incontinence diaper produced by a method according to one of Claims 1-8, **characterized in that** the length of the rear diaper side parts (20) is at least 15 cm, particularly at least 20 cm, more particularly at least 25 cm, more particularly at least 27 cm and more particularly at most 45 cm.

10. Disposable incontinence diaper according to Claim 9, **characterized in that** the length of the rear diaper side parts (20) is at least 10%, particularly at least 15%, more particularly at least 20% and more particularly at least 22%, more particularly at most 40% of the overall length of the disposable incontinence diaper.

11. Disposable incontinence diaper according to Claim 9 or 10, **characterized in that** the front diaper side parts (22) have a shorter longitudinal extent, particularly by at least 5%, more particularly by at least 10%, more particularly by at least 15% and more particularly by at most 30%, than the rear diaper side parts (20).

12. Disposable incontinence diaper according to one or more of Claims 9-11, **characterized in that** the width of the front and/or rear diaper side parts (22, 20) is 10-40 cm, particularly 12-35 cm, more particularly 14-30 cm and more particularly at most 25 cm.

13. Disposable incontinence diaper according to one or more of Claims 9-12, **characterized in that** the front diaper side parts (22) have the same width as the rear diaper side parts (20).

14. Disposable incontinence diaper according to one or more of Claims 9-13, **characterized in that** the lower edge (65) of the rear diaper side parts (20) has a length A and a width B and the convex portion has an extreme point P, wherein the ratio of the length A to the width B of the lower edge (65) of a rear diaper side part (20) is 0.40-0.90 and the ratio of the longitudinal extent E of the extreme point P of the convex portion of a rear diaper side part (20) to the transverse extent F thereof is 0.15-0.80.

15. Disposable absorbent incontinence diaper (2) according to Claim 14, **characterized in that** the ratio of the transverse extent F of the extreme point P of the convex portion of a rear diaper side part (20) to the width B of the lower edge (65) of a rear diaper side part (20) is 0.20-0.60, particularly 0.30-0.50.

16. Disposable absorbent incontinence diaper (2) according to Claim 14 or 15, **characterized in that** the ratio of the longitudinal extent E of the extreme point P of the convex portion of a rear diaper side part (20) to the length A of the lower edge (65) of a rear diaper side part (20) is 0.10-0.40, particularly 0.10-0.30.

17. Disposable absorbent incontinence diaper (2) according to one or more of Claims 14-16, **characterized in that** the ratio of the longitudinal extent E of an extreme point P of the convex portion of a rear diaper side part (20) to the transverse extent F thereof is 0.20-0.60, particularly 0.20-0.50.

18. Disposable absorbent incontinence diaper (2) according to one or more of Claims 14-17, **characterized in that** the ratio of the length A of the lower edge (64) of a rear diaper side part (22) to the width B of the lower edge (64) of a rear diaper side part (22) is 0.50-0.80, particularly 0.55-0.75.

19. Disposable absorbent incontinence diaper (2) according to one or more of Claims 9-18, **characterized in that** the rear diaper side parts (20) have at least one closure means (33) provided with closure aids, in particular with mechanical closure aids.

20. Disposable absorbent incontinence diaper (2) according to one or more of Claims 9-19, **characterized in that** the front and rear diaper side parts (22, 20) are formed from a nonwoven material or comprise a nonwoven material.

## Revendications

1. Procédé de fabrication d'une multiplicité de couches d'incontinence jetables (2) présentant une partie principale de couche (4) et des parties latérales de couche avant et arrière (22, 20) attachées à celle-ci, **caractérisé par** les étapes de procédé suivantes:
- fournir et transporter une bande sans fin de parties principales de couche (50) dans une première direction longitudinale (18) vers une station de coupe et d'application (24),
- fournir une bande sans fin de parties latérales de couche (21) à la station de coupe et d'application (24), dans lequel on découpe à la station de coupe et d'application (24) des premières et des deuxièmes parties longitudinales successives (46a, 46b) à double usage avec des bords au moins localement courbés hors de la bande de parties latérales de couche (21) et on les attache à des premières et deuxièmes régions (56a, 56b) de la bande de parties principales de couche, dans lequel les premières et deuxièmes régions (56a, 56b) comprennent chacune une zone de hanche de deux parties principales de couche (4) se succédant et adjacentes l'une à l'autre en direction longitudinale,
- séparer les couches d'incontinence jetables (2) par coupe de la bande de parties principales de couche (50) transversalement à la première direction longitudinale (18), dans lequel on effectue la coupe à travers les parties longitudinales (46a, 46b) de telle manière qu'une première partie partielle (62a) d'au moins une première partie longitudinale respective (46a) forme une partie latérale arrière de couche (20) d'une première couche d'incontinence jetable et qu'une deuxième partie partielle (64a) de la première partie longitudinale respective (4 6a) forme une partie latérale avant ou arrière de couche (22, 20) d'une deuxième couche d'incontinence jetable directement adjacente, et qu'une première partie partielle (62b) d'une deuxième partie longitudinale respective (46b) forme une partie latérale avant de couche (22) d'une couche d'incontinence jetable et qu'une deuxième partie partielle (64b) de la deuxième partie longitudinale respective (46b) forme une partie latérale avant ou arrière de couche (22, 20) d'une autre couche d'incontinence jetable directement adjacente,
- dans lequel on profile les bords courbés des parties longitudinales (46a, 46b) après la coupe hors de la bande de parties latérales de couche (21) de telle manière qu'après la fixation des premières et des deuxièmes parties longitudinales (46a, 46b) sur les premières et les deuxièmes régions (56a, 56b) de la bande de parties latérales de couche, chaque partie latérale arrière de couche (20) présente un bord inférieur (65), qui présente une partie convexe et chaque partie latérale avant de couche (22) présente un bord supérieur (66), qui présente une partie concave, et que la longueur E d'un bord extérieur (67) d'une partie latérale avant respective de couche (22) soit plus petite que la longueur D d'un bord intérieur (68) d'une partie latérale avant respective de couche (22), et
- dans lequel l'extension longitudinale de la partie latérale avant de couche (22) vaut au moins 60 % de l'extension longitudinale de la partie latérale arrière de couche (20).

2. Procédé selon la revendication 1, **caractérisé en ce qu'**une deuxième partie partielle (64a) de la première partie longitudinale respective (46a) forme une partie latérale arrière de couche (20) et une deuxième partie partielle (64b) de la deuxième partie longitudinale respective (46b) forme une partie latérale avant de couche (22).

3. Procédé selon la revendication 1, **caractérisé en ce qu'**une deuxième partie partielle (64a) de la première partie longitudinale respective (46a) forme une partie latérale avant de couche (22) et une deuxième partie partielle (64b) de la deuxième partie longitudinale respective (46b) forme une partie latérale arrière de couche (22).

4. Procédé selon une ou plusieurs des revendications précédentes, **caractérisé en ce que** l'on effectue la coupe des parties longitudinales (46a, 46b) hors de la bande de parties latérales de couche (21) le long de lignes de coupe (70, 70a, 70b, 70c, 70d, 70e, 71).

5. Procédé selon la revendication 4, **caractérisé en ce qu'**une ligne de coupe (70c) s'étend sous forme sinusoïdale ou ondulée de façon continue à travers la matière de la bande de parties latérales de couche (21) et divise ainsi la bande de parties latérales de couche (21) en deux bandes partielles.

6. Procédé selon la revendication 4, **caractérisé en ce qu'**une ligne de coupe (70, 70a, 70b, 70d) s'étend sous forme sinusoïdale ou ondulée ou en forme d'arc sur un ou deux côté(s) au-delà des bords longitudinaux de la bande de parties latérales de couche (21).

7. Procédé selon une ou plusieurs des revendications précédentes, **caractérisé en ce que** l'on attache des premiers éléments élastiques (80) étendus dans la première direction longitudinale (18) à la bande de parties principales (50).

8. Procédé selon une ou plusieurs des revendications précédentes, **caractérisé en ce que** l'on effectue la fourniture de la bande de parties latérales de couche (21) à la station de coupe et d'application (24) avec une première vitesse v1 et la fourniture de la bande sans fin de parties principales de couche (50) à la station de coupe et d'application (24) avec une deuxième vitesse v2, dans lequel la première vitesse v1 est inférieure à la deuxième vitesse v2.

9. Couche d'incontinence jetable fabriquée par un procédé selon l'une quelconque des revendications 1 à 8, **caractérisée en ce que** la longueur des parties latérales arrière de couche (20) vaut au moins 15 cm, en particulier au moins 20 cm, en particulier encore au moins 25 cm, en particulier encore au moins 27 cm, et en particulier encore au maximum 45 cm.

10. Couche d'incontinence jetable selon la revendication 9, **caractérisée en ce que** la longueur des parties latérales arrière de couche (20) vaut au moins 10 %, en particulier au moins 15 %, en particulier encore au moins 20 % et en particulier encore au moins 22 %, en particulier encore au maximum 40 % de la longueur totale de la couche d'incontinence jetable.

11. Couche d'incontinence jetable selon la revendication 9 ou 10, **caractérisée en ce que** les parties latérales avant de couche (22) présentent une extension longitudinale plus faible, en particulier d'au moins 5 %, en particulier encore d'au moins 10 %, en particulier encore d'au moins 15 % et en particulier encore d'au maximum 30% que les parties latérales arrière (20).

12. Couche d'incontinence jetable selon une ou plusieurs des revendications 9 à 11, **caractérisée en ce que** la largeur des parties latérales avant et/ou arrière de couche (22, 20) vaut 10 à 40 cm, en particulier 12 à 35 cm, en particulier encore 14 à 30 cm, et en particulier encore au maximum 25 cm.

13. Couche d'incontinence jetable selon une ou plusieurs des revendications 9 à 12, **caractérisée en ce que** les parties latérales avant de couche (22) présentent la même largeur que les parties latérales arrière de couche (20).

14. Couche d'incontinence jetable selon une ou plusieurs des revendications 9 à 13, **caractérisée en ce que** le bord inférieur (65) des parties latérales arrière de couche (20) présente une longueur A et une largeur B et la partie convexe présente un point extrême P, dans laquelle le rapport de la longueur A à la largeur B du bord inférieur (65) d'une partie latérale arrière de couche (20) vaut 0,40 à 0,90 et le rapport de l'extension longitudinale E du point extrême P de la partie convexe d'une partie latérale arrière de couche (20) à son extension transversale F vaut 0,15 à 0,80.

15. Couche d'incontinence jetable absorbante (2) selon la revendication 14, **caractérisée en ce que** le rapport de l'extension transversale F du point extrême P de la partie convexe d'une partie latérale arrière de couche (20) à la largeur B du bord inférieur (65) d'une partie latérale arrière de couche (20) vaut 0,20 à 0,60, en particulier 0,30 à 0,50.

16. Couche d'incontinence jetable absorbante (2) selon la revendication 14 ou 15, **caractérisée en ce que** le rapport de l'extension longitudinale E du point extrême P de la partie convexe d'une partie latérale arrière de couche (20) à la longueur A du bord inférieur (65) d'une partie latérale arrière de couche (20) vaut 0,10 à 0,40, en particulier 0,10 à 0,30.

17. Couche d'incontinence jetable absorbante (2) selon une ou plusieurs des revendications 14 à 16, **caractérisée en ce que** le rapport de l'extension longitudinale E d'un point extrême P de la partie convexe d'une partie latérale arrière de couche (20) à son extension transversale F vaut 0,20 à 0,60, en particulier 0,20 à 0,50.

18. Couche d'incontinence jetable absorbante (2) selon une ou plusieurs des revendications 14 à 17, **caractérisée en ce que** le rapport de la longueur A du bord inférieur (64) d'une partie latérale arrière (22) à la largeur B du bord inférieur (64) d'une partie latérale arrière de couche (22) vaut 0,50 à 0,80, en particulier 0,55 à 0,75.

19. Couche d'incontinence jetable absorbante (2) selon une ou plusieurs des revendications 9 à 18, **caractérisée en ce que** les parties latérales arrière de couche (20) présentent au moins un moyen de fermeture (33) muni d'accessoires de fermeture, en particulier d'accessoires de fermeture mécaniques.

20. Couche d'incontinence jetable absorbante (2) selon une ou plusieurs des revendications 9 à 19, **caractérisée en ce que** les parties latérales avant et arrière de couche (22, 20) sont formées d'un matériau non tissé ou comprennent un matériau non tissé.
